(19) Europäisches Patentamt / European Patent Office / Office européen des brevets

(11) **EP 4 200 014 B1**

(12) **EUROPEAN PATENT SPECIFICATION**

(45) Date of publication and mention
of the grant of the patent:
**01.10.2025 Bulletin 2025/40**

(51) International Patent Classification (IPC):
**A61N 5/10** *(2006.01)* **G21K 1/04** *(2006.01)*

(52) Cooperative Patent Classification (CPC):
**A61N 5/1048; A61N 5/1045; A61N 5/1071;
A61N 5/1075;** A61N 2005/1074; G21K 1/046

(21) Application number: **21759413.4**

(22) Date of filing: **17.08.2021**

(86) International application number:
**PCT/IB2021/057573**

(87) International publication number:
**WO 2022/038519 (24.02.2022 Gazette 2022/08)**

(54) **IMAGE-BASED RADIATION THERAPY QUALITY ASSURANCE**

BILDBASIERTE STRAHLENTHERAPIEQUALITÄTSSICHERUNG

ASSURANCE QUALITÉ DE RADIOTHÉRAPIE BASÉE SUR UNE IMAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.08.2020 US 202016999700
19.04.2021 US 202117234653**

(43) Date of publication of application:
**28.06.2023 Bulletin 2023/26**

(73) Proprietor: **Sun Nuclear Corporation
Melbourne, FL 32940 (US)**

(72) Inventors:
• **KAPATOES, Jeffrey M.
Melbourne, Florida 32940 (US)**
• **SIMON, Jeffery
Melbourne, Florida 32940 (US)**
• **TADROUS, Kirolos
Melbourne, Florida 32940 (US)**
• **MCCLASKY, Jason
Melbourne, Florida 32940 (US)**
• **PRICE, Victor
Melbourne, Florida 32940 (US)**
• **WILSON, Ryan
Melbourne, Florida 32940 (US)**

(74) Representative: **Carroll, Christopher P.
Boston & Galway
Golden Cross House
8 Duncannon Street
London WC2N 4JF (GB)**

(56) References cited:
**WO-A1-2016/200463 US-A1- 2008 298 553
US-A1- 2018 140 272**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**DESCRIPTION OF THE RELATED ART**

Radiation therapy can be utilized in the treatment of diseases, for example, by delivering a dose of radiation to kill or to inhibit growth of a cancerous tumor. Devices to deliver radiation therapy can include, for example, radioisotopes, heavy ion accelerators and linear accelerators that generate a photon beam directed at a tumor site. To irradiate a tumor while minimizing exposure to nearby healthy tissues, a radiation beam can be shaped by a collimating device, for example, a multileaf collimator (MLC). Multileaf collimators include a number of movable leaves that can be positioned to create a shaped aperture (e.g., shaped the same as the tumor, from the vantage point of the radiation beam).

Radiation therapy quality assurance can be performed to verify the proper operation of one or more components of a radiation therapy delivery system, for example, verifying the positioning of MLC leaves.

WO2016/200463A1 describes a method that includes determining a first estimate of leaf positions for a plurality of leaves of a multi-leaf collimator (MLC) in a reference coordinate space based on an image generated by an image-based aperture verification system, wherein the leaf positions for the plurality of leaves define an aperture for the MLC. The method further includes determining a second estimate of the leaf positions for the plurality of leaves in the reference coordinate space based on data from an additional source. The method further includes verifying the leaf positions for the plurality of leaves based on comparing the first estimate to the second estimate, wherein the leaf positions are verified if the first estimate deviates from the second estimate by less than a threshold value. US2008/298553A1 describes a particle-beam treatment system in which, even during particle-beam irradiation, the shape of a multileaf collimator is monitored. The particle-beam treatment system, in which multi-layer conformal irradiation is performed while the setting of the shape of the multileaf collimator in an irradiation head is changed during particle-beam irradiation, is provided with an optical shape-monitoring unit mounted attachably and detachably in the snout portion at the downstream side of the collimator, the optical shape-monitoring unit having a shape-monitoring mirror, opposing the multileaf collimator, for monitoring the shape of the multileaf collimator; a video camera for shooting the multileaf-collimator shape reflected by the shape-monitoring mirror; and an image monitor for displaying an image of the video camera that shoots the shape of the multileaf collimator. US2018/140272A1 describes phantom, phantom system, and method of phantom identification that include a first material that forms a phantom. A phantom identifier includes at least one unit marker. The at least one unit marker identifies a physical characteristic of the phantom. In a method of phantom identification, an image of the phantom is obtained that includes the phantom identifier. The at least one unit marker is identified, the at least one unit marker encodes a value representative of a physical characteristic of the phantom.

**SUMMARY**

Systems, computer program products, and methods are disclosed for determining a shape of a radiation field generated by a radiation delivery system that includes a radiation source configured to deliver a radiation beam. An implementation of a system and/or computer program product that determines the shape of the radiation field includes a non-transitory, machine-readable medium storing instructions which, when executed by at least one programmable processor, cause the at least one programmable processor to perform operations comprising: acquiring images capturing at least a portion of a shape representative of a radiation pattern generated by a radiation delivery system that includes a radiation source configured to deliver the radiation beam, wherein a video stream is received and acquiring of the images is performed by extracting the images from the video stream.

Implementations of the current subject matter can include, but are not limited to, methods consistent with the descriptions provided herein as well as articles that comprise a tangibly embodied machine-readable medium operable to cause one or more machines (e.g., computers, etc.) to result in operations implementing one or more of the described features. Similarly, computer systems are also contemplated that may include one or more processors and one or more memories coupled to the one or more processors. A memory, which can include a computer-readable storage medium, may include, encode, store, or the like, one or more programs that cause one or more processors to perform one or more of the operations described herein. Computer implemented methods consistent with one or more implementations of the current subject matter can be implemented by one or more data processors residing in a single computing system or across multiple computing systems. Such multiple computing systems can be connected and can exchange data and/or commands or other instructions or the like via one or more connections, including but not limited to a connection over a network (e.g., the internet, a wireless wide area network, a local area network, a wide area network, a wired network, or the like), via a direct connection between one or more of the multiple computing systems, etc.

The details of one or more variations of the subject matter described herein are set forth in the accompanying drawings and the description below. Other features and advantages of the subject matter described herein will be apparent from the description and drawings, and from the claims. The invention is defined in the claims; other embodiments, aspects, examples etc. not being a part of the invention .

## BRIEF DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings,

Figure 1A is a diagram illustrating a perspective view of a simplified exemplary radiation delivery system in accordance with certain aspects of the present disclosure,

Figure 1B is a diagram illustrating a side-sectional view of the simplified exemplary radiation delivery system of FIG. 1A in accordance with certain aspects of the present disclosure,

Figure 2 is a diagram illustrating a perspective view of a simplified exemplary multileaf collimator shaping a radiation field in accordance with certain aspects of the present disclosure,

Figure 3 is a diagram illustrating views of a simplified exemplary radiation pattern at a scintillator in accordance with certain aspects of the present disclosure,

Figure 3A is a diagram illustrating an exemplary system utilizing a camera to capture images of a radiation pattern displayed on a computer monitor in accordance with certain aspects of the present disclosure,

Figure 4 is a diagram illustrating an end-perspective view of a simplified exemplary scintillator fixed in an orientation that is not perpendicular to the radiation beam axis in accordance with certain aspects of the present disclosure,

Figure 5A is a diagram illustrating a perspective view of a simplified exemplary scintillator and camera fixed in a supporting structure in accordance with certain aspects of the present disclosure,

Figure 5B is a diagram illustrating a perspective view of a simplified exemplary scintillator and camera fixed in a supporting structure that is open on a top and bottom portion in accordance with certain aspects of the present disclosure,

Figure 6 is a diagram illustrating a perspective view of a simplified exemplary combined radiotherapy and medical imaging system in accordance with certain aspects of the present disclosure,

Figure 7 is a diagram illustrating a side-sectional view of the simplified exemplary combined radiotherapy and medical imaging system of FIG. 6 in accordance with certain aspects of the present disclosure,

Figure 8 is a diagram illustrating an end-perspective view down the bore of the simplified exemplary combined radiotherapy and medical imaging system of FIG. 6, showing exemplary scintillators and cameras in accordance with certain aspects of the present disclosure,

Figure 8A is a diagram illustrating an exemplary sequence of how radiation patterns and images thereof may appear at different stages of the disclosed systems, in accordance with certain aspects of the present disclosure,

Figure 9 is a diagram illustrating an exemplary system including a radiation detector in communication with a server for radiation delivery QA in accordance with certain aspects of the present disclosure,

Figure 10 is a diagram illustrating an exemplary method of extracting images from a video stream in accordance with certain aspects of the present disclosure,

Figure 11 is a diagram illustrating an exemplary system including a monitor used with a radiation detector providing a digital video signal that is output to a video encoder in accordance with certain aspects of the present disclosure,

Figure 12 is a diagram illustrating an exemplary system including a console of a radiation detector providing a digital video signal output to a video encoder in accordance with certain aspects of the present disclosure,

Figure 13 is a diagram illustrating an exemplary system including a video encoder providing a video stream to a QA server and providing digital video signal output to a monitor in accordance with certain aspects of the present disclosure,

Figure 14 is a diagram illustrating an exemplary system including a server, which is not part of a radiation detector, providing a video stream to a QA server in accordance with certain aspects of the present disclosure,

Figure 15 is a diagram illustrating an exemplary method of determining leaf positions of a multileaf collimator in accordance with certain aspects of the present disclosure,

Figure 16 is a diagram illustrating an exemplary geometric method of determining a leaf position of a multileaf collimator from a radiation pattern in accordance with certain aspects of the present disclosure.

## DETAILED DESCRIPTION

Radiation therapy quality assurance is a field that includes, among other things, determining whether a radiation delivery system is functioning properly and providing the prescribed radiation dose to a patient as detailed in a radiation therapy treatment plan. While many radiation delivery systems include their own functionality for displaying output and diagnostic metrics, radiation therapy quality assurance products can obtain independent measurements of what the radiation delivery system is providing.

As used herein, the term "radiation delivery system" can include various components needed to generate, direct and control a radiation therapy beam. For example, a radiation therapy system can include a radiation source (e.g. a linear accelerator, particle beam source, etc.), a gantry (fixed or rotating), a collimator (to shape the radiation reaching the patient), imaging equipment (to image prior to or during therapy), and the like.

As part of quality assurance, the operation of various components of the radiation delivery system can be independently assessed. Examples of such operations can include, for example, verifying the output of the radiation source, the position of a rotating gantry, the configuration of a multileaf collimator (e.g., determining its leaf positions), etc. The present disclosure describes, among other things, systems, software, and methods for determining collimator configurations based on the analysis of radiation patterns that emerge after a radiation beam passes through the collimator.

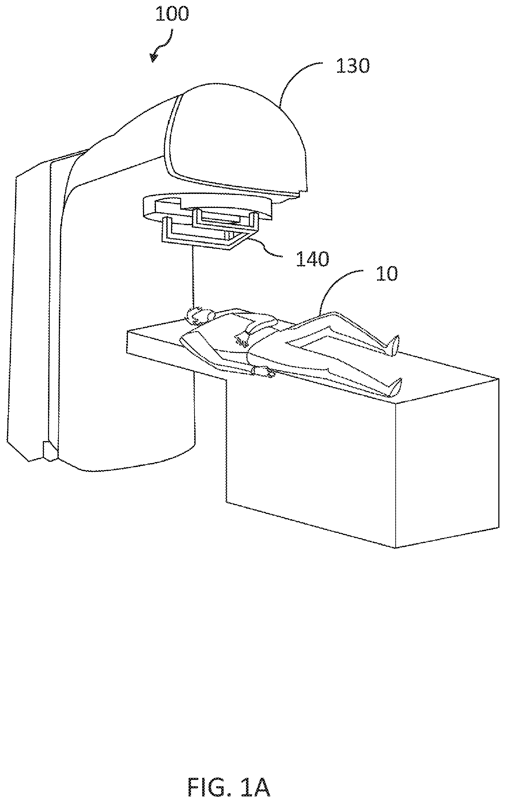

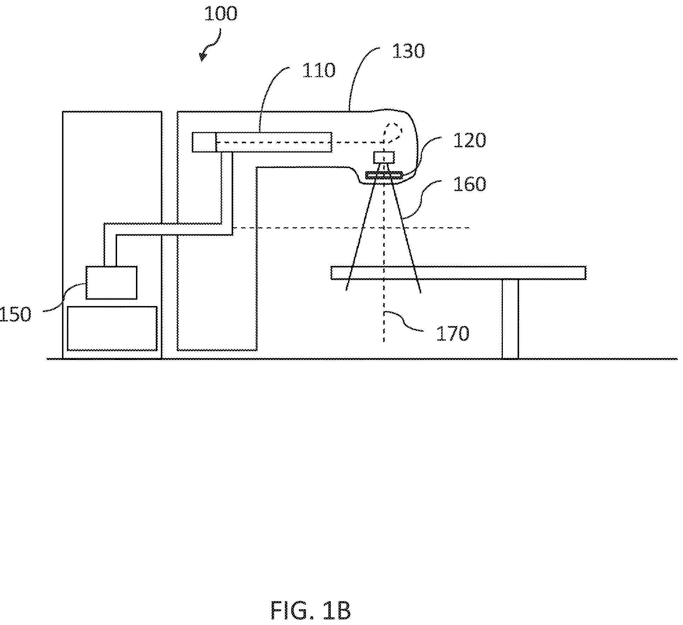

FIGs. 1A and 1B depict an exemplary radiation delivery system 100. This exemplary system is an open (or "C-arm") type system that includes a linear accelerator (e.g., element 110 in FIG. 1B) working with an RF source 150, a multileaf collimator 120, and a rotatable gantry 130. In this exemplary system, the linear accelerator and multileaf collimator are mounted within the rotatable gantry to allow radiation beam 160 to be delivered along beam axis 170 to a patient 10 at multiple angles. FIG. 1A also depicts an accessory tray 140 that can permit the mounting or positioning of hardware or devices between the radiation source and the patient. As described further herein, the technologies of the present disclosure can be used with radiation delivery systems such as the exemplary system depicted in FIGs. 1A and 1B, as well as with other types of radiotherapy systems.

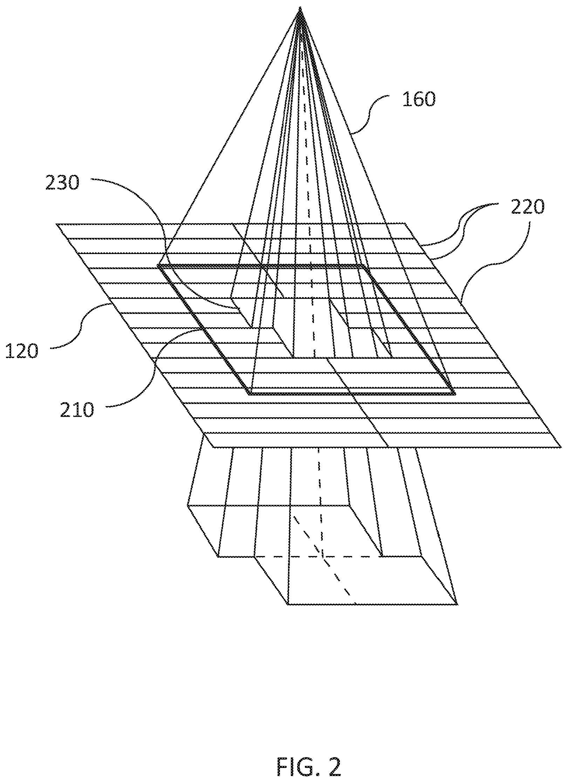

When performing radiation therapy quality assurance, one element of the radiation therapy device that can be assessed is the multileaf collimator (e.g., through verifying the collimator's leaf positions). One method for MLC configuration verification may involve examining the shape of the radiation field delivered to the patient by a radiation delivery system 100 including a radiation source (e.g., linear accelerator 110) configured to deliver a radiation beam 160. As shown in the simplified example of FIG. 2, a radiation field 210 can be shaped by blocking some portions with the leaves 220 of a multileaf collimator 120 to form an aperture 230. The portion of the radiation field that passes through the aperture will then proceed to the patient to deliver a dose of radiation in the desired shape. As used herein, the term "radiation field" can refer to radiation before or after being shaped by a collimator.

Scintillating materials may be used to determine the shape of a radiation field emerging from a multileaf collimator. "Determining the shape" can include determining the overall shape, determining particular MLC leaf positions (which provides information regarding the shape), etc.

"Scintillators," as discussed herein, are understood to include any material that, when hit by radiation, emit radiation (e.g., particle or photon) that can be detected (for example, by a camera). Scintillators include materials that absorb incoming radiation and then re-emit a portion of the absorbed energy in the form of light. It should be noted that when the term "light" is used herein, it is intended to include radiation within, or not within, the visible spectrum (for example, scintillators that emit infrared or other types of radiation are contemplated). Examples of scintillators can include plastic scintillators (such as Li6 plastic scintillators or polyethylene naphthalate), luminophores, crystal scintillators, phosphorescent materials, etc. As used herein, a "camera" can be any device that can detect radiation (e.g., light) coming from a scintillator. Examples of cameras can include CCD cameras, photodiodes, photomultiplier tubes, etc.

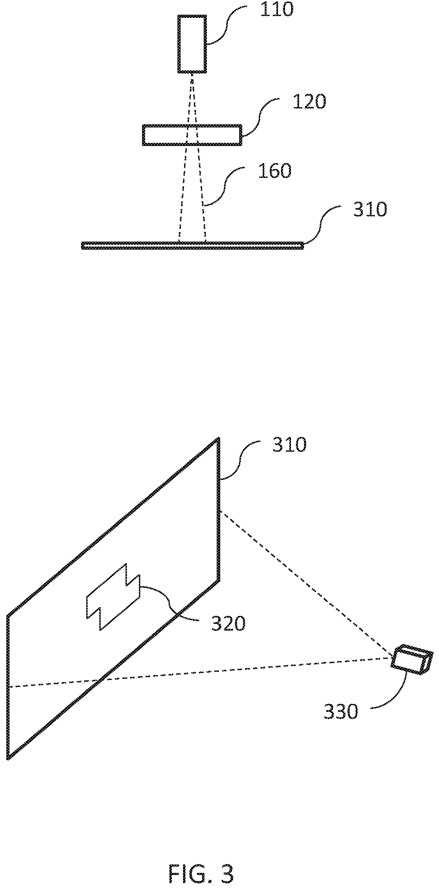

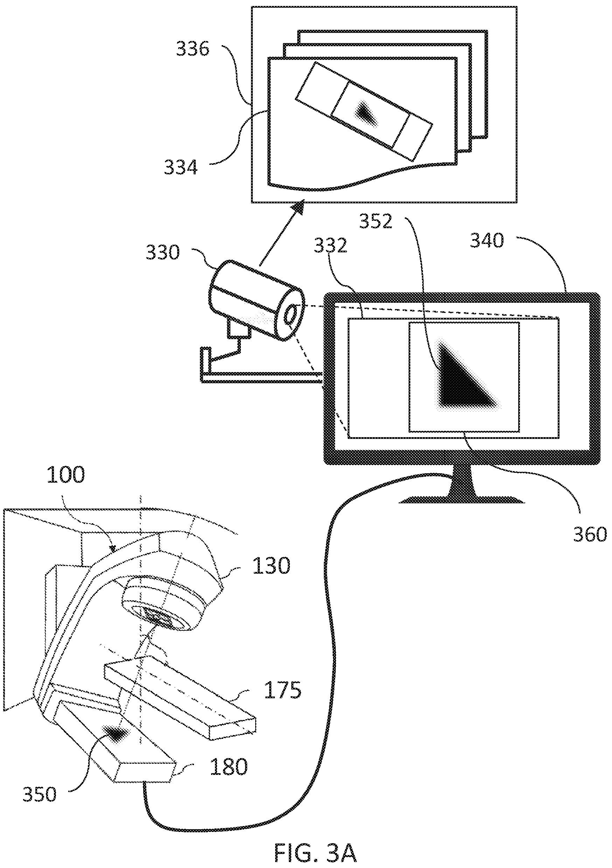

The top portion of FIG. 3 illustrates a simplified example of a scintillator 310 receiving a radiation beam 160 from radiation delivery system 110, after it passes through a multileaf collimator 120.

The lower portion of FIG. 3 illustrates a simplified example of a system for detecting a radiation pattern 320 from scintillator 310 using a camera 330. The radiation pattern 320 is related to the shape of the aperture formed by the multileaf collimator (as used herein, "radiation pattern" refers to the pattern present at the scintillator as it emits radiation/light after exposure to a radiation field).

Analysis of the images or signals acquired by the camera from the scintillator's radiation patterns can provide estimates of leaf positions of the multileaf collimator, independent of leaf position information that may be provided by the radiation delivery system itself.

With reference to Fig. 3a, the disclosure herein relating to obtaining and utilizing a shape of a radiation pattern at a scintillator also applies to obtaining images by, for example, using a camera 330 or screen capturing software to obtain images from a computer monitor 340 displaying information from a radiation detector 180 (e.g., an EPID). Camera 330 can have a field of view 332, which may be different than the extents of the computer monitor. As used herein, and depicted in Fig. 3a, the captured images (e.g., a video file generated by the camera or screen capture software) are referred to as images 334 and can be processed by image processing module 336.

During treatment or quality assurance, personnel may observe at computer monitor 340 the radiation pattern 350 at the radiation detector 180 in terms of an accumulated amount of radiation. Such monitors can display the shape 352 of the radiation pattern at any resolution (i.e., possibly, but not necessarily, the same resolution as the active surface of the radiation detector). For example, the radiation detector could have 1000 x 2000 detector elements but displayed at the computer monitor with 2000 x 4000 pixels, thus corresponding to four pixels at the computer monitor per detector element at the radiation detector. In some implementations, to provide a reference as to the extent of the displayed computer monitor image, the computer monitor may display a bounding box 360 of known size, scale, or other indication of the actual size of the radiation pattern at the radiation detector. Also, the shape can be displayed to also communicate the intensity of radiation. For example, the shape can have pixels with different colors reflecting different intensities or accumulations of radiation. As described further herein, tilted scintillators, computer monitors, or analysis of video streams from the radiation detector can be utilized to provide information about the radiation reaching the radiation detector, with such information usable to derive collimator positions, dose calculation, fluence maps, etc.

The present disclosure describes, among other things, technologies utilizing scintillators to verify collimator leaf positions. However, contrary to what is common in the art, the present disclosure describes certain embodiments that utilize a scintillator that is tilted, so as to not be perpendicular to the axis of the radiation beam. Also contrary to the art, certain embodiments disclose a scintillator system with a very shallow angle between the camera and the scintillator. For example, disclosed systems may be configured such that the angle between a planar scintillator and the camera's line of sight is less than 10 degrees.

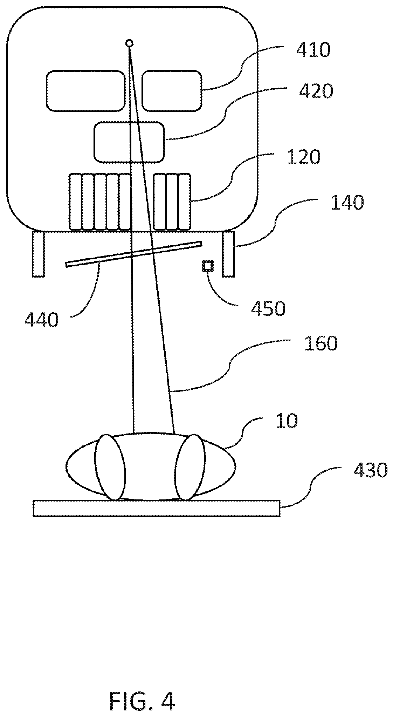

One implementation of the disclosed technology for determining at least a portion of a shape of a radiation field is depicted in FIG. 4. The figure illustrates a simplified representation of a radiation source 402 and a series of collimators (an X-axis jaw 410, a Y-axis jaw 420, and a multileaf collimator 120). Also illustrated is a patient 10 lying on a couch 430 such that the patient is hit by radiation beam 160 after its passes through the collimators. An accessory tray 140 is also depicted between the collimators and the patient couch.

The exemplary system of FIG. 4 can also include a scintillator 440 and a camera 450 configured to acquire images of light emitted by the scintillator during delivery of the radiation beam. As shown in FIG. 4, the scintillator and camera system can be configured to be located between the radiation source and a patient couch, thus functioning as an entrance detector.

It is contemplated that scintillator 440 may include a planar sheet of scintillating material or may include a curved sheet of scintillating material that may, for example, be oriented to have its convex surface facing toward the camera. In some embodiments, scintillator 440 may be sized to be large enough to cover the largest radiation field the radiation delivery system can deliver (at the location of the scintillator). In other embodiments, scintillator 440 may be more compact and may be smaller than the largest radiation field the system can deliver at the location of the scintillator, yet still sufficient for performing some measure of quality assurance.

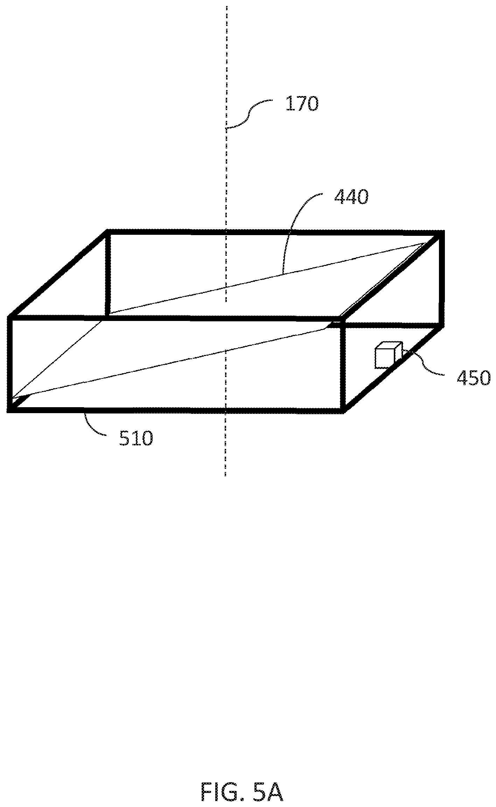

As illustrated in FIG 5A, scintillator 440 and camera 450 can be fixed in a support structure 510. The support structure 510 may then be configured to be mounted to a radiation delivery system so that the scintillator will be struck by the radiation treatment beam.

The scintillator and camera are preferably fixed to the support structure in a manner that sets a specific desired geometric relationship between the scintillator and camera. The exemplary embodiment depicted in FIG. 5A illustrates a simplified framework where the scintillator is located within the support structure and the camera is located and configured to view one side of the scintillator (e.g., its bottom surface). It is contemplated, however, that the camera may be placed in a position to view the top surface of the scintillator or that more than one camera may be used.

In some embodiments, the scintillator and camera can be fixed to the support structure so that when the support structure is mounted to the radiation delivery system, the scintillator is not perpendicular to an axis of the radiation beam. FIG. 4 illustrates such an example of a scintillator 440 not being perpendicular to the axis 170 of radiation beam 160. When referring herein to a scintillator being oriented so that it is not perpendicular to an axis of the radiation beam, such refers to an orientation that is purposefully not perpendicular (i.e., as opposed to an orientation that may slightly deviate from an intended perpendicular positioning).

Some embodiments may have the scintillator fixed to the support structure so that the scintillator will be at an angle of between 80 to 89 degrees or between 91 and 110 degrees relative to the axis of the radiation beam when the support structure is mounted to the radiation delivery system. In other embodiments, the scintillator can be at an angle of between 84 and 88 degrees or between 92 and 96 degrees relative to the axis of the radiation beam when the support structure is mounted to the radiation delivery system. While the exemplary embodiment depicted in FIG. 4 shows a tilt in the Y direction, it is contemplated that a similar tilt could be implemented in the X direction or in both the X and Y directions.

In some embodiments, such as the one depicted in FIG. 5A, camera 450 and scintillator 440 can be fixed to support structure locations that maximize the angle between the camera and the scintillator. In such examples, the camera may be at one end of the support structure, as depicted, or may be in a corner of the support structure, or may even be located outside the perimeter of the support structure. Such embodiments can be beneficial in that the resolution of the radiation pattern imaged by the camera can be increased as compared to smaller angles where the radiation pattern is viewed more edge-on.

The design of the support structure can be substantially open above and/or below the scintillator to reduce or eliminate material that may attenuate the radiation therapy beam. Alternatively, the scintillator and the camera can be substantially enclosed by the support structure (for example, to prevent dust from accumulating on the scintillator or to protect it from damage or scratching). In such embodiments, the top portion and/or bottom portion of the support structure may be designed to provide only minimal attenuation of a radiation beam. For example, the top and/or bottom portion may be a layer of thin plastic or glass that causes only slight attenuation of the radiation beam.

The example depicted in FIG. 5A can represent a support structure 510 that substantially encloses the scintillator (i.e., is closed on all sides), but FIG. 5A is also intended to depict an example where the support structure 510 is merely a frame for mounting the scintillator and camera - with all the sides and the top/bottom being open.

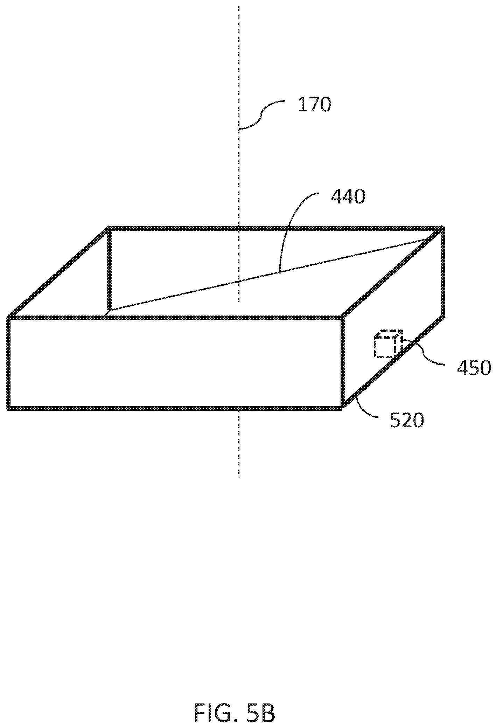

FIG. 5B Illustrates another exemplary embodiment in which the top portion and bottom portion of support structure 520 are open to allow light or other radiation unobstructed access to/through the scintillator, but where the support structure 520 also includes closed structural portions on its sides.

The present disclosure contemplates support structures that are constructed to include any combination of open or closed or transparent/translucent top, bottom or side materials.

Some embodiments of the present disclosure can enable the use of a visible light source (e.g., a tungsten or any sort of atomic lamp, or a white light source) to check the shape of a collimator aperture while the scintillator/support structure is in place. In such embodiments, the support structure can include translucent or transparent portions, which may be, for example, the top portion, bottom portion, or any portion(s) that form the sides of the support structure. It is contemplated that any combination of portions of the support structure may be translucent or transparent. Similarly, the scintillator may also be translucent or transparent. Such translucent or transparent support structure portions and/or scintillators can allow formation of a pattern at a target location (e.g., at the isoplane) corresponding to the shape of a collimator aperture when a light source shines light through the collimator aperture onto the scintillator and/or supporting structure portion. The present disclosure contemplates that any embodiments herein (not just planar scintillator embodiments) can incorporate transparent or translucent support structure portions and/or scintillators.

As used herein, the term "transparent" means that light corresponding to the shape of the collimator aperture is able to pass through without significant distortion, resulting in a pattern that can be accurately related to the shape of the collimator aperture. Similarly, as used herein, the term "translucent" means that light is able to pass, but there may be some distortion or dimming of the light and the resulting pattern corresponding to the shape of the collimator aperture. In embodiments where a translucent material is used, it is contemplated that degree of distortion will not be prohibitive of providing a pattern that can be utilized in radiation therapy quality assurance for determining the shape of a collimator aperture. Also, it is contemplated that the transparent or translucent material described herein can have any degree of attenuation of light. For example, a transparent scintillator may attenuate 50% of light but still allow a sharp (though dimmer) pattern to be formed at the target location. According to the type of application desired, translucent or transparent scintillators can have a polyvinyltoluene base, optionally including some fraction of lead (e.g., approximately 2% - appropriate for x-ray dosimetry), etc.

In some embodiments, the support structure can be configured to be mounted to the radiation delivery system at an accessory tray disposed between the radiation source and a patient couch. For example, a linear accelerator may have an accessory tray or slot into which the support structure may be mounted. It thus contemplated herein that when reference is made to a support structure being "configured to be mounted," this can include, for example, being configured in a way to be removably mounted (e.g., structurally designed to slide into an accessory tray slot or specifically sized to fit within the tray). Support structures herein are also contemplated to be configured to be mounted by virtue of more permanent structures such as the provision of screw holes or other fastening accessories to aid in mounting to a particular portion of a radiation delivery system.

In certain embodiments, the scintillator(s) and camera(s) can be fixed to the support structure in a manner so that the whole assembly fits entirely within an accessory tray.

The support structure mounting, in conjunction with specific fixation therein of the scintillator and camera can result in a tilted scintillator orientation with regard to the axis of the radiation beam. For example, mounting the support structure into a litizic accessory tray that is perpendicular to the axis of the radiation beam, when the scintillator is fixed at an angle within the supporting structure, results in the scintillator being tilted with regard to the axis of the radiation beam.

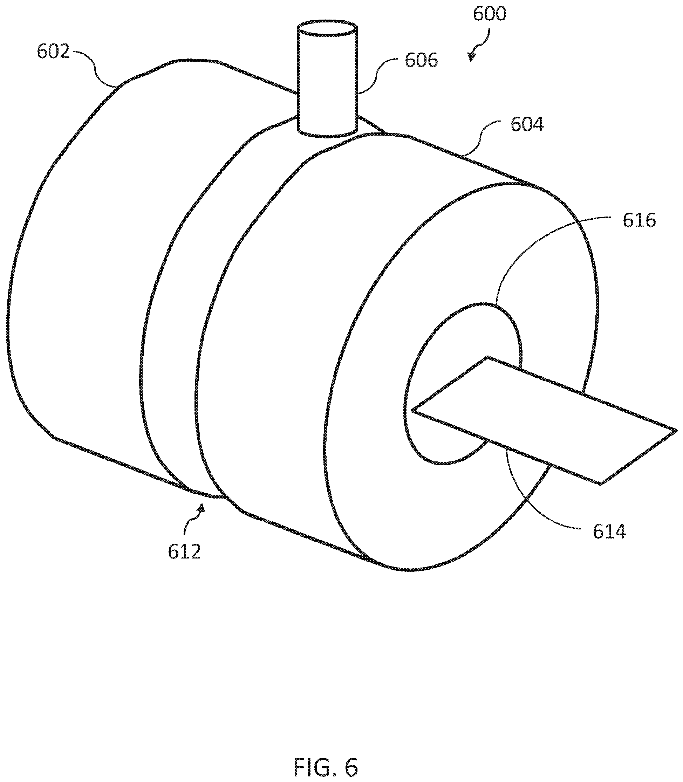

In contrast to the scintillator/camera systems described above for a C-arm type radiation delivery system, the present disclosure contemplates alternative embodiments for implementation with radiation delivery systems having a bore, for example, a radiation delivery system combined with an imaging system such as an MRI. FIG. 6 shows a simplified example of such a system 600 that includes first and second magnet housings 602 and 604 separated by gap region 612. A radiation source 606 can be mounted to a gantry adjacent to or in a gap region 612. A patient can be positioned on couch 614 inside bore 616 of the magnet so that the gantry can cause rotation of radiation source 606 around the patient.

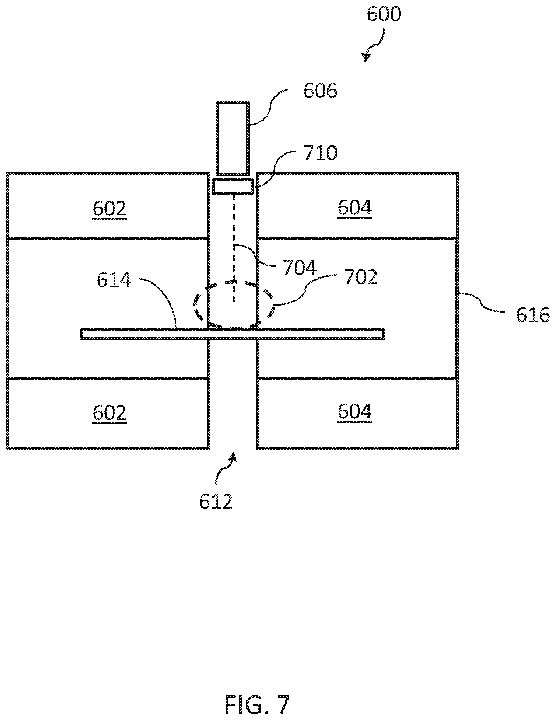

FIG. 7 depicts a cross-sectional view of the exemplary system shown in FIG. 6. Such a system can be used to image a target region 702 (e.g., a volume and/or area within a patient), while radiation source 606 emits radiation 704 for patient treatment. Also shown is a multileaf collimator 710 for shaping the radiation beam directed toward the patient. The system illustrated in FIGs. 6 and 7 is only one example of a radiotherapy system having a bore that is compatible with embodiments of the present disclosure. Implementations of the technologies herein may also be used with other types of radiotherapy systems that include a patient bore.

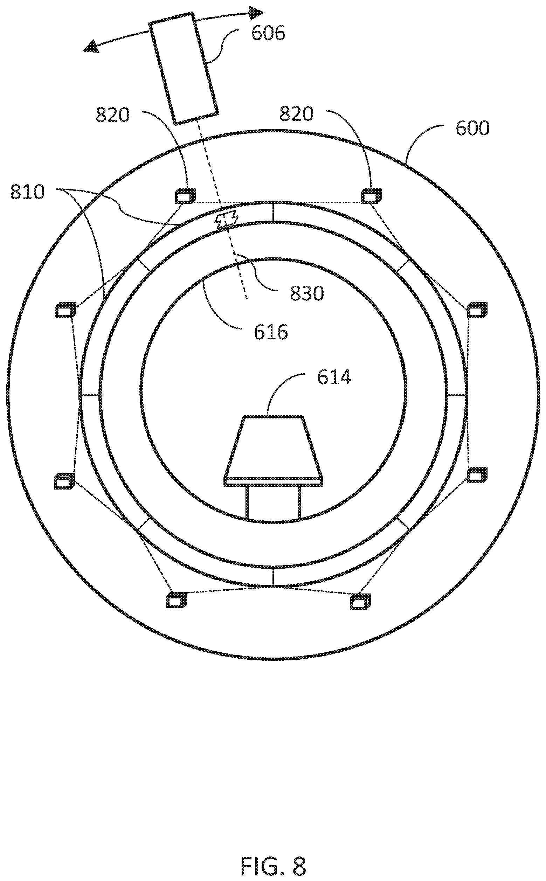

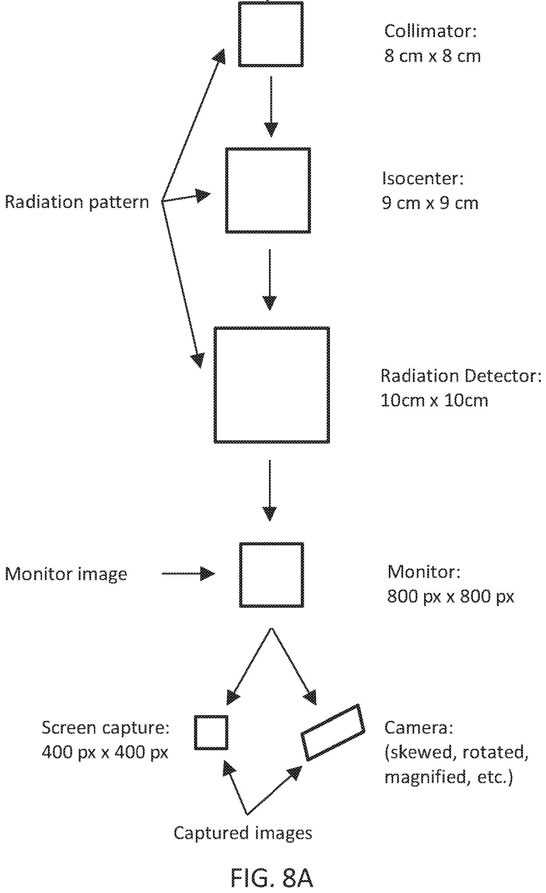

Scintillators that are shaped or configured for radiotherapy systems having a bore may be utilized in certain implementations. For example, as shown FIG. 8, scintillator 810 can be mounted to radiation delivery system 600 such that scintillator 810 substantially follows the contour of a bore 616 of a radiation delivery system 600. As noted above with regard to FIGs. 6 and 7, the bore may be part of an MRI-guided radiotherapy system. "Substantially following the contour of a bore" is understood to include, for example, following the internal surface of the bore or having generally the same contour as the bore but a slightly larger or smaller diameter, etc.

The scintillator may be a continuous sheet of scintillating material or may be comprised of multiple sheets. The example of FIG. 8 illustrates an exemplary scintillator having multiple curved sheets of scintillating material, specifically, eight curved sheets, each covering 45 degrees of the bore. In other implementations, the scintillator may be made up of a number of planar sheets of scintillating material. For example, the configuration shown in FIG. 8 could instead be comprised of eight planar sheets, meeting at their edges. It is contemplated that any number of curved or planar sheets can be implemented, in any feasible combination, to cover a desired portion of the bore and they can be located at any radial distance from the axis of the bore.

While the scintillator can extend around the entire circumference of the bore, it is not essential that it do so. For example, the scintillator can cover any degree or angular measure of the bore (e.g., 270 degrees, 180 degrees, 90 degrees, 45 degrees, etc.) and may be constituted of any number of sheets (e.g., ten sheets covering 27 degrees each, 18 sheets covering 10 degrees each, etc.).

As described in further detail herein, mounting the scintillator to the radiation delivery system may include, for example: mounting the scintillator directly to a portion of the radiation delivery system such as the gantry, the linac, the MLC, etc.; mounting the scintillator to the bore of an imaging system associated with the radiation delivery system (e.g., an MRI for an MRI-guided radiation therapy system); and, mounting the scintillator indirectly, for example, mounting the scintillator to a supporting structure that can in turn be mounted to portions of the overall system (e.g., RT device, MRI, etc.).

In some embodiments, the scintillator can be mounted so it is at an angle to the radiation beam or the scintillator can be mounted so that at least one portion of the scintillator remains perpendicular to axis 830 of the radiation beam when the radiation source 606 is controlled to move around the bore. For example, in instances where the radiation beam axis 830 is radial and the scintillator is curved to be concentric with the bore, at least one portion of the scintillator (e.g., where axis 830 intersects the scintillator) would be perpendicular to axis 830.

One or more cameras configured to acquire images of light emitted by the scintillator during delivery of the radiation beam can be utilized. Similar to the previously-described embodiments, these may be mounted so as to have a shallow angle between the scintillator and the camera. For example, the cameras can be configured to be mounted at an angle of greater than 0 and less than 10 degrees relative to the scintillator. In other embodiments, the cameras may be mounted to result in angles of 10-20, 20-30, 30-40, 40-50 or 50-60 degrees between the scintillator and the line of sight of the camera.

Cameras can be placed on the bore at various locations so they are able to view at least a portion of the scintillator. The cameras can be small so as to provide minimal intrusion into the inner volume of the bore where the patient is located. As shown in the example of FIG. 8, each of the scintillator sections or sheets may have a corresponding camera with a field of view covering it (shown approximately by the dotted lines). The number and disposition of the cameras and their fields of view in FIG. 8 are examples only and other configurations are contemplated. For example, the fields of view can cover only a portion of a section rather than an entire section, the cameras can be located at any axial location along the bore, and they can be on either side of the scintillator.

In some embodiments, a support structure can be configured to be mounted to the bore, and the one or more cameras can be fixed to the support structure. In the present example, a support structure configured to be mounted to the bore may include a cylindrical framework that generally conforms to the shape of the bore, such that the supporting structure having the cameras can be inserted or installed in the bore, without the need to mount individual cameras to the bore structure itself. In such embodiments, and other embodiments, such as those described above with reference to FIG. 8, the cameras can be oriented to view respective portions of the scintillator adjacent to the cameras. Here, the scintillator "adjacent the camera" means the scintillator can be located at least partially at the same angular location as the camera. In addition to mounting cameras on such a support structure, other embodiments can include having the scintillator also mounted to the support structure. Such embodiments can allow for the entire assembly of scintillators and cameras to be inserted or mounted to the bore as a unit, rather than as individual components.

In other embodiments, the camera can be mounted to view a portion (i.e., some or all) of a radiation pattern displayed at a computer monitor showing radiation that was delivered to the radiation detector. The camera can be located at any position, for example, attached to the computer monitor via a mounting arm, mounted to a table or wall near the computer monitor, etc. As such, the camera can have any viewing angle relative to the computer monitor.

Accordingly, the disclosure of the present, and parent, applications contemplate, among other things, the general concepts of acquiring images capturing at least a portion of a shape representative of a radiation pattern generated by a radiation delivery system that includes a radiation source configured to deliver a radiation beam.

Thus, in addition to utilizing a scintillator to obtain images, the images can be of a computer monitor of a radiation detector, the operations further comprising determining one or more dimensions of the radiation pattern based on determining a conversion between the images and computer monitor images of the radiation pattern.

As explained further below, the present and parent disclosures thus also contemplate the utilization of the captured images and the calibration techniques described herein during treatment or as part of quality assurance, to perform, for example, dose calculation, collimator position determination (e.g., MLC leaf position), fluence determinations, etc. The captured images can be acquired from the camera aimed at a computer monitor displaying the shape that is representative of the radiation pattern. The camera may be mounted in a fixed relationship to the computer monitor by mounting to the computer monitor itself or another location nearby. To allow for a user to be in front of the monitor, it is contemplated that in some implementations the images can be acquired at an angle not perpendicular to the computer monitor. In some implementations, the camera can be fixed to the computer monitor so that the camera will be at an angle of between 1 and 10 degrees relative to a screen of the computer monitor (with 90 degrees being perpendicular to the screen). In other implementations, the camera can be is fixed to the computer monitor so that the camera will be at an angle of between 4 and 8 degrees relative to the screen of the computer monitor. In some implementations to have a more direct viewing of the computer monitor, the camera can be fixed at a location that maximizes the angle between the camera and the screen. For example, the location can be a wall generally opposite the monitor.

FIG. 8A illustrates an example of how the size of a radiation pattern may vary when captured by a camera viewing a computer monitor (or by screen capture at the computer monitor). The dimensions provided in the example are only for illustrative purposes and are not to scale. For example, a collimator can be controlled to form an aperture of 8 cm x 8 cm. Due to beam divergence and based on distances along the beam axis, this aperture can result in a radiation pattern of 9 cm x 9 cm at the isocenter and 10 cm x 10 cm further along at the radiation detector. The radiation pattern, as displayed at the computer monitor (i.e., the "computer monitor image," could be 800 pixels x 800 pixels. If a screen capturing technique is used, the captured images of the radiation pattern could be, for example, 400 pixels x 400 pixels. Similarly, if using a camera, due to factors including distance, offset, rotation, viewing angle, etc., the captured images from the camera could be rotated, skewed, magnified, offset, etc. When the images are corrected in software, they can then have the same shape as the radiation pattern (e.g., square), but with their final size in pixels depending on the final transformations used. However, with corrections/conversions, the images can be used to derive dimensions of radiation patterns and/or collimator positions, etc.

The present disclosure provides several methods for determining dimensions of a radiation pattern, positions of a collimator used to shape the radiation field, etc. As described herein, computer monitor images may be captured by a camera or with screen capture software. As previously illustrated in Fig. 8A, a complicating factor can be that the camera (and screen capture software) may generate images having a different resolution. Thus, the present disclosure provides implementations of methods and software algorithms to establish a conversion for the camera or screen capture images to provide a measure of the actual dimensions of the radiation pattern at the radiation detector (or other useful locations). Thus, in general, the software that performs this conversion (also referred to as "image processing module") can determine a conversion between the captured images and the radiation pattern.

In some implementations, the image processing module can receive conversion information entered by a user after measuring the geometric relationship between the camera and the computer monitor. In other implementations, conversion information can be determined based on utilizing imaging of markers placed at known locations. In yet other implementations, conversion information can be determined that establishes a relationship between image intensity and delivered dose.

In other embodiments, the relationship between the pixels size of the displayed image and the radiation detector can be established by the original equipment manufacturer (OEM) *a priori.* In such circumstances, this calibration process can become a quality assurance process to confirm this relationship is as stated by the OEM.

Information for conversion of camera images received by the image processing module can include: camera angle (which can introduce a different conversion of the horizontal (X) and vertical (Y) pixels in the camera image), distance between the camera and the computer monitor, magnification of the images, offset between the center of the camera's FOV and the center of the viewing field at the computer monitor (i.e., the center of the camera image of the computer monitor not coinciding with the location at the radiation detector of the axis of the radiation beam), the angle of the camera, etc. Other factors that can be considered are the refresh rate of the computer monitor, the frame rate of the camera, either (or both) of which can result in image blurring or missing data. In this way, the operations for determining the conversion can include applying one or more of a scaling, rotation, or skew correction to the images.

Described below are exemplary methods for use with a camera imaging the radiation detector's computer monitor. Then, other exemplary methods are described for directly capturing the output of the computer monitor without the use of a camera.

First, a predefined radiation field can be created that has known dimensions. For example, a collimator can be controlled to have an aperture 8 cm x 8 cm. The camera can then acquire images of the resultant radiation pattern at a scintillator or from the computer monitor. In software, the conversion between pixels in the camera image and the known size of the aperture can be established as a calibration for the camera images. Thus, when imaging a radiation field of unknown or varying dimensions, this calibration can be applied to convert the camera images into actual dimensions of the aperture. Similarly, with a known beam divergence and distance from the collimator to the radiation detector, isocenter, or any other location, the images can then be converted or used to measure the radiation field at those locations as well.

In another implementation, a graticule or other structure having markers representing known distance(s) and/or having known thicknesses can be placed at any location (e.g., on the radiation detector, at the isocenter, on the scintillator, etc.) and imaged. The markers can attenuate the beam and aid in determining image calibrations, as described further below.

In some implementations, acquiring of images can be performed by screen capture of the computer monitor displaying the shape representative of the radiation pattern. Such implementations have advantages in that additional hardware (e.g., a camera) is not required, which eliminates error that could be introduced by uncertainty in a camera angle or position. Described below are some factors that can be implemented in determining conversions utilizing images acquired through screen capture.

In one implementation, the conversion can be based on a ratio of pixels in the computer monitor images to the pixels in the acquired images. In another implementation, calibration methods similar to those discussed above can be performed where a radiation pattern of known dimension is projected onto the radiation detector. With known dimensions of the radiation pattern in pixels in the captured images, a conversion factor can be established. Thus, determining the conversion can include applying a scaling (likely) or rotation (if applicable). The scaling or rotation can utilize information entered by a user.

In some implementations, the acquiring of images can be performed through utilization of video capture techniques applied to video outputs of the radiation detector. The term radiation detector in this case refers not only to the detector itself (e.g., an EPID panel) but also to its associated electronics, which may include a computer console, a monitor, etc., that are used in conjunction with an EPID panel to process and display the radiation patterns detected by the EPID.

Some radiation detectors can generate output in the form of "digital video signal output." As used herein, the term "digital video signal output" and other forms of the term encompass output from the radiation detector (e.g., a video of the measured radiation pattern) that can be displayed at a dedicated monitor of the radiation detector or electronically transmitted over a connection for, e.g., storage or display at a remote system/monitor. The digital video signal output can be provided from the radiation detector via a variety of physical connections. For example, the digital video signal output may come from an HDMI port, an ethernet or RJ45 connector, etc. The formatting of the digital video signal output can determine how it may be used and if further processing is needed.

Similar to other implementations described herein, the images acquired from the digital video signal output (e.g., acquired during or after delivery of a radiation beam) can capture (e.g., represent/contain) at least a portion of a shape representative of a radiation pattern generated by a radiation delivery system that includes a radiation source configured to deliver the radiation beam.

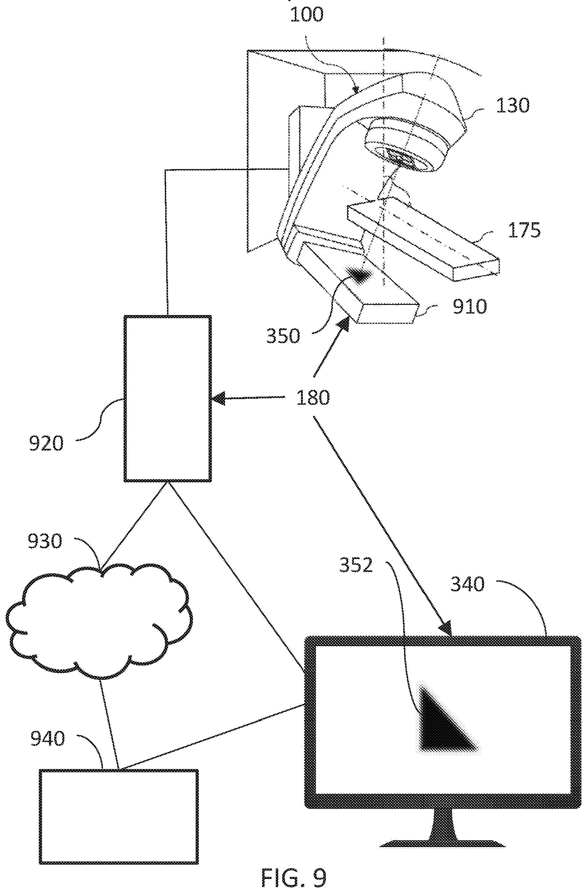

As used herein, a radiation detector may be any sort consistent with the present disclosure (e.g., any configured to measure a radiation pattern). One example of a radiation detector 180 can include an EPID as shown in FIGs. 3A and 9. FIG. 9 depicts an implementation where radiation detector 180 can include EPID panel 910 for receiving the radiation, console 920 that processes data from the EPID panel, and monitor 340 for displaying the shape 352 of radiation pattern 350. In general, panel 910 can refer to any radiation-sensitive portion of a radiation detector, console 920 can refer to any computing systems associated with processing data from panel 910 and monitor 340 can refer to any display device for showing data associated with the radiation detector.

Another example of a radiation detector may be a scintillator coupled to an array of photomultiplier tubes or a scintillator imaged with a camera. Output of the array or the camera can be digitized to also be digital video signal output, similar to the EPID radiation detector example. Accordingly, no specific type of radiation detector (e.g., an EPID) is required or essential for use with the image/video capture concepts disclosed herein.

Some implementations of radiation detector 180 can include console 920 producing a digital video signal output formatted for display on monitor 340. The digital video signal output can than later be provided as a video stream to a Quality Assurance (QA) server 940. Other implementations can, additionally or alternatively, provide a video stream at QA server 940 that is received over network 930 without being relayed through monitor 340. The acquisition of images useful for radiation therapy QA can also include extracting images from a video stream received at QA server 940. As used herein, the term "video stream" encompasses data formatted such that it can be transmitted/received over a network connection. Examples of formats for a video stream can include, for example, H.264, H.265, RTMP, RTSP, UDP, HTTP, M3U8, or Multicast SRT.

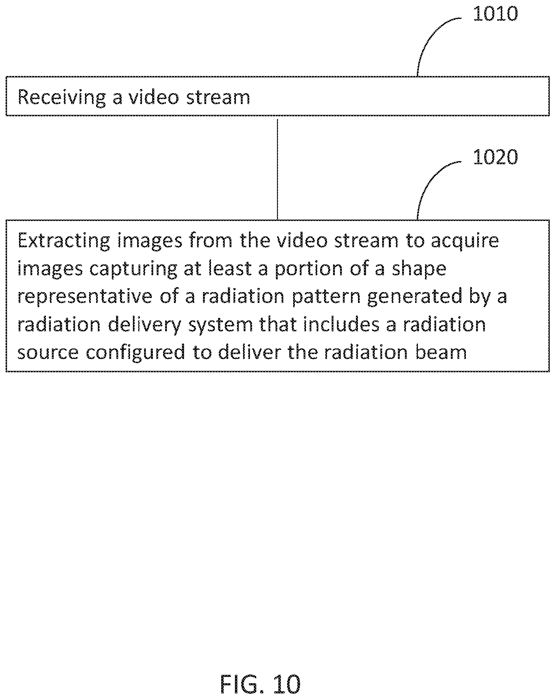

As illustrated by the flow chart in FIG. 10, computer software can perform operations to acquire images for QA by extracting them from a video stream. In the exemplary implementation of FIG. 10, at 1010, a video stream can be received. The receipt of the video stream can be at, for example, QA server 940 but may also be at any other associated computing systems that may perform preprocessing or other operations on the video stream before delivery to the QA server. For example, the video stream may go through routers, encryption/decryption hardware, etc., before arrival at the QA server.

At 1020, images can be extracted from the video stream in several different ways to acquire images capturing at least a portion of a shape representative of a radiation pattern generated by a radiation delivery system that includes a radiation source and beam limiting devices configured to deliver and shape the radiation beam. Image extraction can be performed, for example, by isolating individual video frames from the video stream and saving the video frames as image files. In another implementation, digital interpolation can be used to generate frames at time indices between those of the closest frames (such as when the video frame rate is lower than desired). For example, if the video frame rate was 10 frames per second (fps) but 30 frames per second were needed, two intermediate frames can be generated for each frame based on digitally interpolating adjacent frames in the video stream. In another implementation, digital averaging can be used on a comparatively high frame rate video stream. For example, if the video stream corresponded to a frame rate of 120 fps but 30 fps is needed, four frames can be digitally averaged (e.g., averaging pixel intensities) to generate a single frame. Other implementations can include processing such as skipping some frames, e.g., to sparsely sample the video stream, digitally averaging overlapping groups of frames, or otherwise smoothing the video stream, removing artifacts resulting from video capture, or calculating differences between frames or images from different moments in time. For example, some radiation detectors acquire and accumulate image data over a given acquisition period and thus any given frame is actually an accumulation of image data. In such cases, earlier frames may be subtracted from later frames.

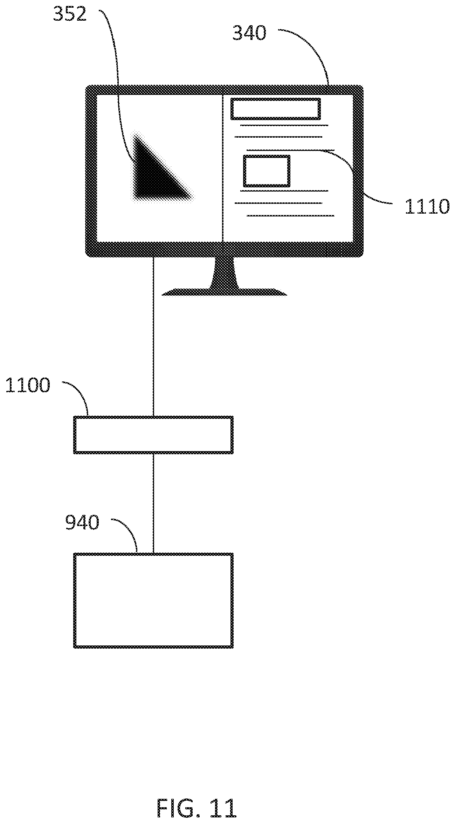

FIG. 11 illustrates utilizing a video encoder as part of the video capture process. In this implementation, a digital video signal output can be provided to a video encoder 1 100 by monitor 340 that is associated with the radiation detector. The monitor can do so via an HDMI port, DVI port, etc. In the example of Fig. 11, the monitor depicts the display of shape 352 of the radiation pattern on the left half of the screen and additional information 1110 (e.g., patient name, ID, or other data) on the right half of the screen. As used herein, when monitor 340 is said to be "associated with" the radiation detector, this means that the monitor is specifically provided for displaying output from the radiation detector. For example, the monitor may be a screen integrated into the radiation detector or a dedicated peripheral that comes with the console and panel. Alternatively, the monitor may be any sort of monitor that has been integrated with the radiation detector for the display of information.

Video encoder 1100 can receive the digital video signal output from the monitor at an input port (e.g., HDMI, DVI, etc.). The video encoder can then convert/encode the digital video signal output into a video stream. The video stream can then be output from the video encoder via a LAN connection or other connection such that it may be streamed to computing systems for performing radiation therapy QA (e.g., QA server 940). In this way, the converting (or encoding) can take the digital video signal output, which can initially be in a format suitable only for direct video display at a monitor and encode it into a format suitable for transmission over network connections. The converted or encoded digital video signal output is then considered to be a "video stream." Video stream formats can include, for example, MPEG, WebM, AVI, MOV, Flash Video, etc.

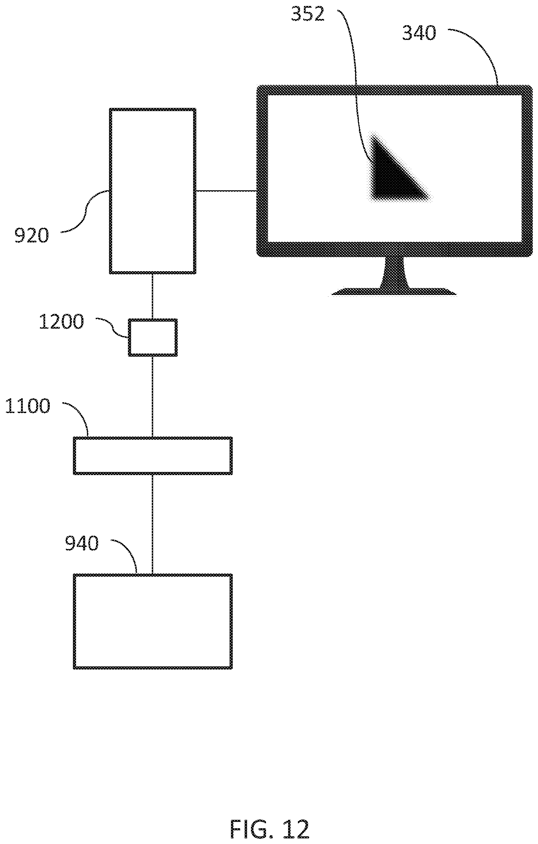

FIG. 12 illustrates a video encoder receiving a digital video signal output from a radiation detector. In general, rather than requiring that the digital video signal output be received specifically from a monitor, the digital video signal output can be received at the video encoder from any component of the radiation detector. For example, the digital video signal output can be received from the console of the radiation detector. As used herein, the term "console" generally refers to computers or other hardware associated with the radiation detector but generally excluding the panel and the monitor. The console can provide the digital video signal output via, for example, an ethernet or RJ45 connector, an HDMI port, etc. In some implementations, a physical converter 1200 can be utilized to convert an ethernet or RJ45 connector to an HDMI port or other connectors such as VGA, DVI, DisplayPort, etc., which can then be connected to the video encoder via a corresponding type of cable. However, the use of such a converter 1200 is not essential. For example, in other implementations such as ones where video encoder 1100 may already have an ethernet or RJ45 connector, the connection between console and the video encoder can be made directly without the use of a converter. Once the digital video signal output is received by the video encoder, the video encoder can convert the digital video signal output to the video stream, as described herein. The video stream can then be streamed to QA server 940 or another connected system.

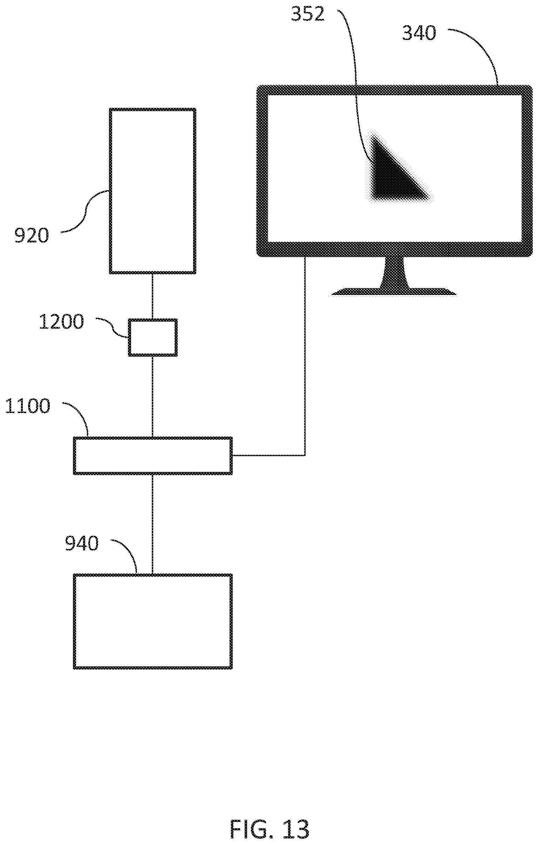

FIG. 13 illustrates a video encoder 1100 additionally sending a digital video signal output to a monitor 340. In implementations where the monitor is part of a radiation detector, the disclosed system can be configured to transmit the digital video signal output from the video encoder to a monitor associated with the radiation detector. For example, the video encoder can have an HDMI output port that can be connected to a monitor with an HDMI input port. Such implementations may be utilized to provide uninterrupted video display of information from the radiation detector.

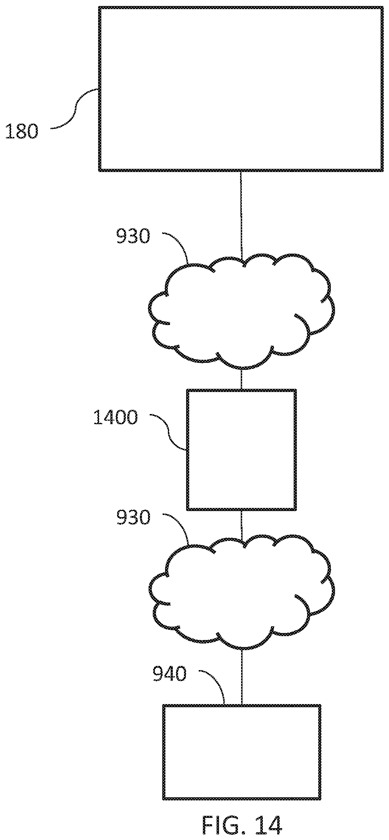

FIG. 14 illustrates an example of a QA server 940 receiving a video stream from a server 1400 that is not part of the radiation detector 180. In some implementations, the video stream can be received from a server 1400 that is not part of the radiation detector. When referring to a server "that is not part of" the radiation detector, this means that server 1400 would not include processors in panel 910, console 920, or monitor 340. One example of server 1400 is a remote computing system, connected to network(s) 930, for relaying a video stream version of the digital video signal output. The remote computing system can be, for example, part of a hospital's computing system that provides video from the radiation detector to physicians. In some implementations, the digital video stream can be displayed live/in real-time such as radiation is detected and imaged by the radiation detector.

For QA server 940 to receive the video stream, one implementation can include QA server 940 connecting to server 1400 via a website link or URL. The QA server can then download the video stream from server 1400. Processing of the video stream into video files and/or images useful for radiation therapy QA can then proceed as described herein.

In another implementation, the system can obtain a recorded video stream from the video stream obtained from server 1400. For example, while the previous implementation contemplated that such streaming may be "live" or "real-time," other implementations can utilize a recording of a previously acquired video stream such that the acquired images utilized for radiation therapy QA can be obtained from the recorded video stream. In some implementations, the recorded video stream can be accessed from a video archive. Such an archive can include ones organized, for example, by patient, timestamp, or other identifier. In implementations where the recorded video stream is in the format of video files or images that can be used without the encoding needed for a video stream, such a recorded video stream can be directly usable by QA server 940.

In some implementations, for example, where the use of a video encoder is not needed due to the radiation detector providing a usable video stream, a recorded video stream can be generated from a video stream originating from the radiation detector. In this way, the acquired images used for radiation therapy QA can be obtained from the recorded video stream, rather than requiring a video encoder or real-time analysis.

In other implementations, start and end points of the recorded video stream can be determined by analyzing the video stream to determine when the patient changes. For example, individual patients may be identified in the video stream by a patient name or identification number. QA server 940 can analyze the video stream to identify the patient (e.g., determining/analyzing portions of video frames in locations where the name is displayed) and isolate and/or extract one or more frames or video segments from the entire received video stream for use in QA analysis. Other identifying information can also be utilized, for example, timestamps that are known to be associated with a patient. In yet other implementations, rather than restricting a segment of a video stream broadly to a patient, a similar process can be implemented (e.g., based on a treatment fraction or timestamp) to obtain start and end times corresponding to a particular portion of the treatment delivery for the patient. For example, start and end times for treatment of a tumor at a given gantry angle may be obtained, intermediate video stream data may then be ignored until another start time is determined, and then the start and end times for treatment of the tumor at a different gantry angle can be obtained. Similar methods can be used outside patient treatment, such as during commissioning or calibration where radiation may be delivered to a phantom as part of radiation delivery QA.

Based on the present disclosure relating to use of cameras, screen capture software, and video stream capture hardware/software to obtain images of and calculate dimensions associated with a radiation pattern, the following example of use is provided. One such method can include the following steps (though not limited to the order shown below):

Step 1 - placing a graticule with markers that have known thicknesses and/or known dimensions between the markers. The markers can be made of one material or of different materials of similar or varying radiation attenuation. In this way, the markers will be visible in images due to being opaque to delivered radiation.

Step 2 - initiating delivery of a radiation beam.

Step 3 - imaging the graticule with the radiation detector (e.g., an EPID) during delivery of the radiation beam.

Step 4 - acquiring images during the delivery of the radiation beam, the images capturing at least a portion of the graticule (e.g., from screen capture, a computer monitor with a camera aimed at it, or video stream capture).

Step 5 - determining a conversion factor based on at least the known dimensions of the graticule and the acquired images.

In some embodiments, the shapes of the radiation pattern (e.g., EPID images) may be displayed on the screen along with additional information 1110. One example of this is shown in Fig. 11, depicting an exemplary shape 352 on the left half of the screen. Thus, in embodiments where the video capture is performed by screen capture, video camera, HDMI out of a monitor, etc., it is understood that additional processing steps may be required in order to identify the location "on the screen" of the EPID image.

As shown in the example of FIG. 11, the software controlling the display of the images may cause the pane with shape 352 to not occupy the full screen. For example, the software may have a preset display configuration or allow for resizing the pane. Accordingly, some implementations can include auto-detecting elements of interest, such as the location of the borders of the pane containing the shape, locating other visual markers in the image display such as the patient name and ID, etc. Autodetection can be performed by direct reading if such display data is available in a data file or embedded in the video stream. In embodiments where such display data is not available, image analysis tools such as edge or object detection (e.g., of displayed border lines or text) can be utilized to determine the locations of the borders or selected visual markers. In this way, the additional information 1110 may be disregarded and the boundaries of the area containing shape 352 can be accurately determined. Similarly, determining a patient name/ID can be performed no matter where they are presented on the display.

Methods and software that enable the determination of MLC leaf positions are disclosed herein. Leaf positions can be reflected in scintillator radiation patterns imaged by one or more cameras, obtained via screen capture software, or obtained via video stream capture hardware/software, as described above. In one embodiment, leaf position determination can be facilitated by analyzing the edges of radiation patterns. As used herein, "radiation pattern" means the image of (or data representing the image of) a) scintillator light emitted due to interaction between the scintillator and a radiation beam and/or b) electronic data representing radiation as measured by a radiation detector.

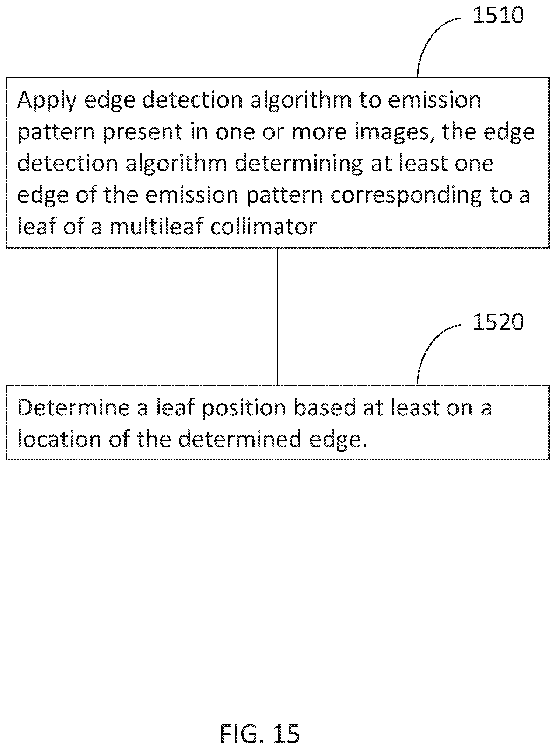

As illustrated by the example in FIG. 15, computer software (e.g., executed at QA server 940) can perform operations to determine edges of a radiation pattern. For example, at 1510, using the image data acquired from the camera(s) and/or video stream(s), an edge detection algorithm (e.g., a Canny edge detection algorithm) can be applied to a radiation pattern present in the images. The edge detection algorithm can determine at least one edge of the radiation pattern corresponding to a leaf of a multi-leaf collimator. From this, at 1520, a leaf position can be determined based at least on a location of the determined edge. Following this determination, it is possible to compare leaf positions during delivery of the radiation beam with planned leaf positions (e.g., as dictated by a radiation therapy plan and/or detailed in system log files). The comparison can thus be utilized in radiation therapy quality assurance.

The process of determining leaf positions can then include, for example, compensating for image distortion caused by (or inherently present in) a given camera or camera system. For example, lens aberrations, and camera placement with respect to the scintillator can be accounted for. One method of accounting for optical effects from the camera system can include performing a calibration procedure with a well-known pattern that allows for a mapping of points in the image acquired by the camera to real positions in the object plane (e.g., the plane of a planar scintillator sheet). This correction/mapping can be performed for any number of opposing leaves of the MLC.

As part of radiation therapy quality assurance, it can be desired to determine leaf positions at a plane through the isocenter. One method for doing so can include determining the effective size of an opening between collimator leaves at a plane parallel to the isocenter plane. Then, the effective MLC leaf positions at the isocenter plane can be determined based on the effective size, when geometrically extended to the isocenter plane.

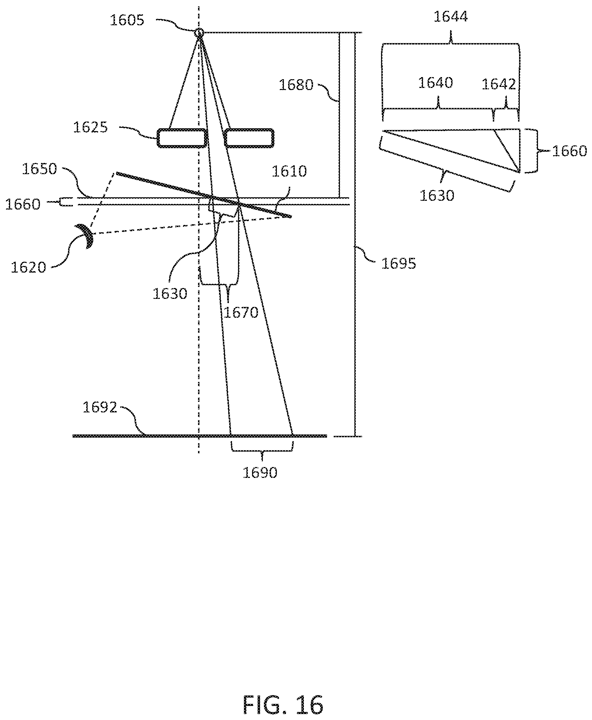

An exemplary arrangement of a simplified system used for the above determination is illustrated in FIG. 16. Here, an example of a tilted scintillator 1610 is shown with a camera 1620 imaging the scintillator 1610. The radiation from radiation source 1605 passing through the MLC 1625 results in a radiation pattern at the scintillator. The shape of the radiation pattern is a length ($length_{screen}$) 1630 that corresponds to the size of the opening between MLC leaves (which can be directly related to the position of the MLC leaves). One exemplary formula for determining the length ($length_{pp}$) 1640 at on a plane parallel 1650 to the isocenter plane 1692 can be expressed as shown in Eq. 1, below.

$$length_{pp} = \sqrt{length_{screen}^2 - height^2} + \frac{x}{(d_1 + height)}$$

$$length_{pp} = \sqrt{length_{screen}^2 - height^2} + height \frac{x}{(d_1 + height)}. \qquad (1)$$

In Eq. 1, the *height* is the height 1660 of the radiation pattern as measured in the vertical direction (or parallel to the beam axis). *X* is the *X*-coordinate 1670 of the right edge of the radiation pattern. *d1* (element 1680) is the height from the radiation source to the plane parallel to the isocenter plane. The expanded view on the right side of FIG. 16 illustrates that $length_{pp}$ 1640 can be found by accounting for the vertical projection 1642 of the radiation pattern to the plane parallel 1650. Once $length_{pp}$ is found the length ($length_{iso}$) 1690 at the isocenter plane 1692 can be determined according to the following relation:

$$length_{iso} = \frac{d_2}{d_1} length_{pp}. \qquad (2)$$

In Eq. 2, $d_2$ (element 1695) is the distance from the radiation source to the isocenter plane 1692. The above description and solution of the simplified geometrical arrangement of the scintillator and camera system should not be considered limiting or exclusive of other solutions that may be implemented for embodiments described herein. Furthermore, it is readily apparent that the above disclosure for a flat scintillator can apply to any flat surface or its equivalent, for example, the above-described computer monitor or image files obtained via screen capture of the computer monitor.

The methods and operations described herein can further enable the determination of fluence maps at the isocenter plane, which can be useful for performing radiation therapy quality assurance. For example, the present disclosure contemplates software that can perform operations that include calculating a fluence map based at least on the leaf positions determined using the scintillator and also on beam output data obtained from the radiation therapy system. Furthermore, operations such as calculating a dose at a target location based at least on the fluence map and a patient image obtained from an imaging system may also be performed. Fluence maps, dose calculations, collimator shapes/MLC leaf positions, and other quantities that can be derived with the benefit of the disclosure herein are also described in commonly owned patent applications: U.S. Patent Application 14/694,865 (now U.S. Patent No. 10,617,891) "Radiation detector Calibration" and U.S. Patent Application 15/395,852 "Determination Of Radiation Collimator Component Position,"

Below is one example of a method of calibrating the disclosed radiation detector monitoring system to allow accurate determination of delivered dose at the radiation detector. This example allows the user to establish a relationship between the dose delivered by the RT system and the intensity of the pixels as seen on the monitor and the image acquired by the radiation detector. The exemplary method can include any or all of the following steps, not all of which need be performed in the order shown.

Step 1 - The user can deliver a series of fixed (e.g., square or rectangular) radiation patterns, each pattern at a different dose level.

Step 2 - For each radiation pattern, the user can enable recording of the monitor screen by the camera or screen capturing via the software interface to the radiation detector system.

Step 3 - The user can turn the beam off and stop the camera or screen capturing software.

Step 4 - The camera or screen capturing software can write out a file containing a video of the screen during image acquisition.

Step 5 - The user can open the video file and measure the pixel intensity via software. This can be a statistical measure such as the average or median intensity in an area of the radiation pattern.

Step 6 - The user can enter the dose level for that radiation pattern thus establishing a relationship between the pixel intensity and the delivered dose from the radiation therapy system.

Step 7 - The user can repeat this process for all fields referred to in Step 1.

Step 8 - The dose calibration can then be saved for use during treatment or later quality assurance. In some implementations, the method may be used in conjunction with the processes described in U.S. Patent Application 14/694,865 (now U.S. Patent No. 10,617,891) and U.S. Patent Application 15/395,852.

A further example of use is provided, describing a clinical treatment workflow. The exemplary method can include any or all of the following steps, not all of which need be performed in the order shown.

Step 1 - The user can set the patient up for treatment and ready the radiotherapy delivery system for treatment.

Step 2 - The user can manually start video acquisition of the monitor by the camera or screen capturing software. Acquisition may occur continuously, and an image processing module (i.e., a collection of software operations and processors utilized for image processing, such as QA server 940) automatically processes the captured video files into segments in which the beam was being delivered at various points in the treatment.

Step 3 - The user can initiate beam delivery and the RT delivery system can deliver all treatment beams to the patient.

Step 4 - Treatment ends and the user can stop video acquisition of the monitor by the camera or screen capturing software.

Step 5 - The camera or screen capturing software can write out a file containing a video of the monitor during image acquisition.

Step 6 - The image processing system can automatically process the video file. This can be achieved by a software routine that monitors the folder in which the video files are saved.

Step 6a - Alternately, the user can manually transfer the video file to a predefined location or opens the video file directly in the image processing system.

Step 7 - The image processing system inputs the video file into a software module (e.g., an image processing module) containing the algorithm as defined herein or by either of U.S. Patent Application 14/694,865 (now U.S. Patent No. 10,617,891) and U.S. Patent Application 15/395,852. The software module can compute the leaf positions as function of time. This information can be used to generate a DICOM RT Plan object that can be used for dose computation.

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" (or "computer readable medium") refers to any computer program product, apparatus and/or device, such as for example magnetic discs, optical disks, memory, and Programmable Logic Devices (PLDs,), used to provide machine instructions and/or data to a programmable processor, including a machine-readable medium that receives machine instructions as a machine-readable signal. The term "machine-readable signal" (or "computer readable signal") refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like.

## Claims

1. A computer program product comprising a non-transitory, machine-readable medium storing instructions which, when executed by at least one programmable processor, cause the at least one programmable processor to perform operations comprising:

   acquiring images capturing at least a portion of a shape representative of a radiation pattern generated by a radiation delivery system that includes a radiation source configured to deliver a radiation beam; and
   receiving a video stream, where the acquiring is performed by extracting the images from the video stream.

2. The computer program product of claim 1, the operations further comprising:

   receiving, at a video encoder, a digital video signal output from a monitor associated with a radiation detector; and
   converting, with the video encoder, the digital video signal output to the video stream.

3. The computer program product of claim 1, the operations further comprising:

   receiving, at a video encoder, a digital video signal output from a radiation detector; and
   converting, with the video encoder, the digital video signal output to the video stream.

4. The computer program product of claim 1, wherein the video stream is received from a server that is not part of a radiation detector.

5. The computer program product of claim 1, the operations further comprising:

   generating a recorded video stream from the received video stream, wherein the acquired images are obtained from the recorded video stream; and
   determining start and end points of the recorded video stream by analyzing the video stream to determine when the patient changes.

6. The computer program product of claim 1, the operations further comprising:

   applying an edge detection algorithm to a radiation pattern present in the images, the edge detection algorithm determining at least one edge of the radiation pattern corresponding to a leaf of a multi-leaf collimator; and
   determining a leaf position based at least on a location of the determined edge.

7. The computer program product of claim 6, the operations further comprising comparing the leaf position with a planned leaf position, the comparing utilized in radiation therapy quality assurance.

8. The computer program product of claim 7, the operations further comprising calculating a fluence map based at least on the leaf position and beam output data obtained from the radiation therapy system.

9. The computer program product of claim 8, the operations further comprising calculating a dose at a target location based at least on the fluence map and a patient image obtained from an imaging system.

10. A method comprising:

    receiving a video stream; and
    acquiring images from the video stream, the images capturing at least a portion of a shape representative of a radiation pattern generated by a radiation delivery system that includes a radiation source configured to deliver the radiation beam,
    wherein the acquiring is performed by extracting the images from the video stream.

11. The method of claim 10, further comprising:

    receiving, at a video encoder, a digital video signal output from a monitor associated with a radiation detector; and
    converting, with the video encoder, the digital video signal output to the video stream.

12. The method of claim 10, further comprising:

    receiving, at a video encoder, a digital video signal output from a radiation detector; and
    converting, with the video encoder, the digital video signal output to the video stream.

13. The method of claim 10, wherein the video stream is received from a server that is not part of a radiation detector.

14. The method of claim 10, further comprising:

    generating a recorded video stream from the received video stream, wherein the acquired images are obtained from the recorded video stream; and
    determining start and end points of the recorded video stream by analyzing the video stream to determine when the patient changes.

15. A computer program product comprising a non-transitory, machine-readable medium storing instructions which, when executed by at least one programmable processor, cause the at least one programmable processor to perform operations comprising:
    acquiring images during delivery of a radiation beam, the images capturing at least a portion of a shape representative of a radiation pattern generated by a radiation delivery system that includes a radiation source configured to deliver the radiation beam, wherein the images are acquired from a camera aimed at a computer monitor displaying the shape representative of the radiation pattern.

16. The computer program product of claim 15, wherein the camera is mounted in a fixed relationship to the computer monitor by mounting to the computer monitor itself or to another location nearby, and the images are acquired at an angle not perpendicular to the computer monitor.

17. The computer program product of claim 16, the operations further comprising:

    receiving conversion information entered by a user after measuring a geometric relationship between the camera and the computer monitor, or
    determining conversion information based on utilizing imaging of markers placed at known locations.

18. A computer program product comprising a non-transitory, machine-readable medium storing instructions which, when executed by at least one programmable processor, cause the at least one programmable processor to perform operations comprising:
    acquiring images during delivery of a radiation beam, the images capturing at least a portion of a shape representative of a radiation pattern generated by a radiation delivery system that includes a radiation source configured to deliver the radiation beam, wherein the acquiring is performed by screen capture of a computer monitor displaying the shape representative of the radiation pattern.

19. The computer program product of claim 15 or 18, the operations further comprising:

applying an edge detection algorithm to a radiation pattern present in the images, the edge detection algorithm determining at least one edge of the radiation pattern corresponding to a leaf of a multi-leaf collimator; and determining a leaf position based at least on a location of the determined edge.

**Patentansprüche**

1. Computerprogrammprodukt, umfassend ein nichttransitorisches, maschinenlesbares Medium, das Anweisungen speichert, die, wenn sie von wenigstens einem programmierbaren Prozessor ausgeführt werden, den wenigstens einen programmierbaren Prozessor veranlassen, Operationen durchzuführen, umfassend:

Erfassen von Bildern, die wenigstens einen Teil einer Form erfassen, die für ein von einem Strahlenabgabesystem erzeugtes Strahlenmuster repräsentativ ist, das eine zur Abgabe eines Strahlenbündels konfigurierte Strahlenquelle beinhaltet; und
Empfangen eines Videostreams, wobei das Erfassen durch Extrahieren der Bilder aus dem Videostreams durchgeführt wird.

2. Computerprogrammprodukt nach Anspruch 1, wobei die Operationen ferner umfassen:

Empfangen, an einem Video-Encoder, eines digitalen Videosignalausgangs von einem Monitor, der einem Strahlendetektor zugeordnet ist; und
Umwandeln, mit dem Video-Encoder, des digitalen Videosignalausgangs in den Videostream.

3. Computerprogrammprodukt nach Anspruch 1, wobei die Operationen ferner umfassen:

Empfangen, an einem Video-Encoder, eines digitalen Videosignalausgangs von einem Strahlendetektor; und
Umwandeln, mit dem Video-Encoder, des digitalen Videosignalausgangs in den Videostream.

4. Computerprogrammprodukt nach Anspruch 1, wobei der Videostream von einem Server empfangen wird, der nicht Teil eines Strahlendetektors ist.

5. Computerprogrammprodukt nach Anspruch 1, wobei die Operationen ferner umfassen:

Erzeugen eines aufgezeichneten Videostreams aus dem empfangenen Videostream, wobei die erfassten Bilder aus dem aufgezeichneten Videostream erhalten werden; und
Bestimmen von Start- und Endpunkten des aufgezeichneten Videostreams durch Analysieren des Videostreams, um zu bestimmen, wann der Patient sich ändert.

6. Computerprogrammprodukt nach Anspruch 1, wobei die Operationen ferner umfassen:

Anwenden eines Kantenerkennungsalgorithmus auf ein in den Bildern vorhandenes Strahlenmuster, wobei der Kantenerkennungsalgorithmus wenigstens eine Kante des Strahlenmusters bestimmt, die einer Lamelle eines Multilamellenkollimators entspricht; und
Bestimmen einer Lamellenposition, die wenigstens auf einem Ort der bestimmten Kante basiert.

7. Computerprogrammprodukt nach Anspruch 6, wobei die Operationen ferner Vergleichen der Lamellenposition mit einer geplanten Lamellenposition umfassen, wobei das Vergleichen bei der Strahlentherapiequalitätssicherung genutzt wird.

8. Computerprogrammprodukt nach Anspruch 7, wobei die Operationen ferner Berechnen einer Fluenzkarte umfassen, die wenigstens auf der Lamellenposition und von dem Strahlentherapiesystem erhaltenen Strahlausgangsdaten basiert.

9. Computerprogrammprodukt nach Anspruch 8, wobei die Operationen ferner Berechnen einer Dosis an einem Zielort umfassen, die wenigstens auf der Fluenzkarte und einem von einem Bildgebungssystem erhaltenen Patientenbild basiert.

10. Verfahren, umfassend:

Empfangen eines Videostreams; und
Erfassen von Bildern aus dem Videostream, wobei die Bilder wenigstens einen Teil einer Form erfassen, die für ein von einem Strahlenabgabesystem erzeugtes Strahlenmuster repräsentativ ist, das eine zur Abgabe des Strahlenbündels konfigurierte Strahlenquelle beinhaltet,
wobei das Erfassen durch Extrahieren der Bilder aus dem Videostream durchgeführt wird.

11. Verfahren nach Anspruch 10, ferner umfassend:

Empfangen, an einem Video-Encoder, eines digitalen Videosignalausgangs von einem Monitor, der einem Strahlendetektor zugeordnet ist; und
Umwandeln, mit dem Video-Encoder, des digitalen Videosignalausgangs in den Videostream.

12. Verfahren nach Anspruch 10, ferner umfassend:

Empfangen, an einem Video-Encoder, eines digitalen Videosignalausgangs von einem Strahlendetektor; und
Umwandeln, mit dem Video-Encoder, des digitalen Videosignalausgangs in den Videostream.

13. Verfahren nach Anspruch 10, wobei der Videostream von einem Server empfangen wird, der nicht Teil eines Strahlendetektors ist.

14. Verfahren nach Anspruch 10, ferner umfassend:

Erzeugen eines aufgezeichneten Videostreams aus dem empfangenen Videostream, wobei die erfassten Bilder aus dem aufgezeichneten Videostream erhalten werden; und
Bestimmen von Start- und Endpunkten des aufgezeichneten Videostreams durch Analysieren des Videostreams, um zu bestimmen, wann der Patient sich ändert.

15. Computerprogrammprodukt, umfassend ein nichttransitorisches, maschinenlesbares Medium, das Anweisungen speichert, die, wenn sie von wenigstens einem programmierbaren Prozessor ausgeführt werden, den wenigstens einen programmierbaren Prozessor veranlassen, Operationen durchzuführen, umfassend:
Erfassen von Bildern während der Abgabe eines Strahlenbündels, wobei die Bilder wenigstens einen Teil einer Form erfassen, die für ein von einem Strahlenabgabesystem erzeugtes Strahlenmuster repräsentativ ist, das eine zur Abgabe des Strahlenbündels konfigurierte Strahlenquelle beinhaltet, wobei die Bilder von einer Kamera erfasst werden, die auf einen Computermonitor gerichtet ist, der die für das Strahlenmuster repräsentative Form anzeigt.

16. Computerprogrammprodukt nach Anspruch 15, wobei die Kamera in einer festen Beziehung zum Computermonitor montiert ist, indem sie am Computermonitor selbst oder an einer anderen Stelle in der Nähe montiert wird, und die Bilder in einem nicht senkrechten Winkel zum Computermonitor erfasst werden.

17. Computerprogrammprodukt nach Anspruch 16, wobei die Operationen ferner umfassen:

Empfangen von Umwandlungsinformationen, die von einem Benutzer nach dem Messen einer geometrischen Beziehung zwischen der Kamera und dem Computermonitor eingegeben wurden, oder
Bestimmen von Umwandlungsinformationen basierend auf der Nutzung der Bildgebung von an bekannten Orten platzierten Markern.

18. Computerprogrammprodukt, umfassend ein nichttransitorisches, maschinenlesbares Medium, das Anweisungen speichert, die, wenn sie von wenigstens einem programmierbaren Prozessor ausgeführt werden, den wenigstens einen programmierbaren Prozessor veranlassen, Operationen durchzuführen, umfassend:
Erfassen von Bildern während der Abgabe eines Strahlenbündels, wobei die Bilder wenigstens einen Teil einer Form erfassen, die für ein von einem Strahlenabgabesystem erzeugtes Strahlenmuster repräsentativ ist, das eine zur Abgabe des Strahlenbündels konfigurierte Strahlenquelle beinhaltet, wobei das Erfassen durch Bildschirmaufnahme eines Computermonitors durchgeführt wird, der die für das Strahlenmuster repräsentative Form anzeigt.

**19.** Computerprogrammprodukt nach Anspruch 15 oder 18, wobei die Operationen ferner umfassen:

Anwenden eines Kantenerkennungsalgorithmus auf ein in den Bildern vorhandenes Strahlenmuster, wobei der Kantenerkennungsalgorithmus wenigstens eine Kante des Strahlenmusters bestimmt, die einer Lamelle eines Multilamellenkollimators entspricht; und
Bestimmen einer Lamellenposition, die wenigstens auf einem Ort der bestimmten Kante basiert.

## Revendications

**1.** Produit de programme informatique comprenant un support non transitoire, lisible par machine, stockant des instructions qui, lorsqu'elles sont exécutées par au moins un processeur programmable, amènent l'au moins un processeur programmable à réaliser des opérations comprenant :

l'acquisition d'images capturant au moins une partie d'une forme représentative d'un diagramme de rayonnement généré par un système de distribution de rayonnement qui comporte une source de rayonnement configurée pour délivrer un faisceau de rayonnement ; et
la réception d'un flux vidéo, où l'acquisition est réalisée en extrayant les images à partir du flux vidéo.

**2.** Produit de programme informatique selon la revendication 1, les opérations comprenant également :

la réception, au niveau d'un encodeur vidéo, d'un signal vidéo numérique émis par un moniteur associé à un détecteur de rayonnement ; et
la conversion, avec l'encodeur vidéo, du signal vidéo numérique émis vers le flux vidéo.

**3.** Produit de programme informatique selon la revendication 1, les opérations comprenant également :

la réception, au niveau d'un encodeur vidéo, d'un signal vidéo numérique émis par un détecteur de rayonnement ; et
la conversion, avec l'encodeur vidéo, du signal vidéo numérique émis vers le flux vidéo.

**4.** Produit de programme informatique selon la revendication 1, dans lequel le flux vidéo est reçu à partir d'un serveur qui ne fait pas partie d'un détecteur de rayonnement.

**5.** Produit de programme informatique selon la revendication 1, les opérations comprenant également :

la génération d'un flux vidéo enregistré à partir du flux vidéo reçu, dans lequel les images acquises sont obtenues à partir du flux vidéo enregistré ; et
la détermination des points de début et de fin du flux vidéo enregistré en analysant le flux vidéo pour déterminer quand le patient change.

**6.** Produit de programme informatique selon la revendication 1, les opérations comprenant également :

l'application d'un algorithme de détection de bord à un diagramme de rayonnement présent dans les images, l'algorithme de détection de bord déterminant au moins un bord du diagramme de rayonnement correspondant à une feuille d'un collimateur multi-feuilles ; et
la détermination d'une position de feuille sur la base d'au moins un emplacement du bord déterminé.

**7.** Produit de programme informatique selon la revendication 6, les opérations comprenant également la comparaison de la position de feuille avec une position de feuille planifiée, la comparaison étant utilisée dans l'assurance qualité de radiothérapie.

**8.** Produit de programme informatique selon la revendication 7, les opérations comprenant également le calcul d'une carte de fluence sur la base au moins de la position de feuille et des données d'émission du faisceau obtenues à partir du système de radiothérapie.

**9.** Produit de programme informatique selon la revendication 8, les opérations comprenant également le calcul d'une dose à un emplacement cible sur la base au moins de la carte de fluence et d'une image de patient obtenue à partir d'un système d'imagerie.

**10.** Procédé comprenant :

la réception d'un flux vidéo ; et
l'acquisition d'images à partir du flux vidéo, les images capturant au moins une partie d'une forme représentative d'un diagramme de rayonnement généré par un système de distribution de rayonnement qui comporte une source de rayonnement configurée pour délivrer le faisceau de rayonnement,
dans lequel l'acquisition est réalisée en extrayant les images à partir du flux vidéo.

**11.** Procédé selon la revendication 10, comprenant également :

la réception, au niveau d'un encodeur vidéo, d'un signal vidéo numérique émis par un moniteur associé à un détecteur de rayonnement ; et
la conversion, avec l'encodeur vidéo, du signal vidéo numérique émis vers le flux vidéo.

**12.** Procédé selon la revendication 10, comprenant également :

la réception, au niveau d'un encodeur vidéo, d'un signal vidéo numérique émis par un détecteur de rayonnement ; et
la conversion, avec l'encodeur vidéo, du signal vidéo numérique émis vers le flux vidéo.

**13.** Procédé selon la revendication 10, dans lequel le flux vidéo est reçu à partir d'un serveur qui ne fait pas partie d'un détecteur de rayonnement.

**14.** Procédé selon la revendication 10, comprenant également :

la génération d'un flux vidéo enregistré à partir du flux vidéo reçu, dans lequel les images acquises sont obtenues à partir du flux vidéo enregistré ; et
la détermination des points de début et de fin du flux vidéo enregistré en analysant le flux vidéo pour déterminer quand le patient change.

**15.** Produit de programme informatique comprenant un support non transitoire, lisible par machine, stockant des instructions qui, lorsqu'elles sont exécutées par au moins un processeur programmable, amènent l'au moins un processeur programmable à réaliser des opérations comprenant :
l'acquisition d'images pendant la délivrance d'un faisceau de rayonnement, les images capturant au moins une partie d'une forme représentative d'un diagramme de rayonnement généré par un système de distribution de rayonnement qui comporte une source de rayonnement configurée pour délivrer le faisceau de rayonnement, dans lequel les images sont acquises à partir d'une caméra visant un moniteur d'ordinateur affichant la forme représentative du diagramme de rayonnement.

**16.** Produit de programme informatique selon la revendication 15, dans lequel la caméra est montée dans une relation fixe par rapport au moniteur d'ordinateur en étant montée sur le moniteur d'ordinateur lui-même ou sur un autre emplacement à proximité, et les images sont acquises à un angle non perpendiculaire au moniteur d'ordinateur.

**17.** Produit de programme informatique selon la revendication 16, les opérations comprenant également :

la réception d'informations de conversion saisies par un utilisateur après avoir mesuré une relation géométrique entre la caméra et le moniteur d'ordinateur, ou
la détermination des informations de conversion sur la base de l'utilisation de l'imagerie de marqueurs placés à des emplacements connus.

**18.** Produit de programme informatique comprenant un support non transitoire, lisible par machine, stockant des instructions qui, lorsqu'elles sont exécutées par au moins un processeur programmable, amènent l'au moins un processeur programmable à réaliser des opérations comprenant :
l'acquisition d'images pendant la délivrance d'un faisceau de rayonnement, les images capturant au moins une partie d'une forme représentative d'un diagramme de rayonnement généré par un système de distribution de rayonnement qui comporte une source de rayonnement configurée pour délivrer le faisceau de rayonnement, dans lequel l'acquisition est réalisée par la capture d'écran d'un moniteur d'ordinateur affichant la forme représentative du diagramme de rayonnement.

19. Produit de programme informatique selon la revendication 15 ou 18, les opérations comprenant également :

l'application d'un algorithme de détection de bord à un diagramme de rayonnement présent dans les images, l'algorithme de détection de bord déterminant au moins un bord du diagramme de rayonnement correspondant à une feuille d'un collimateur multi-feuilles ; et
la détermination d'une position de feuille sur la base d'au moins un emplacement du bord déterminé.

FIG. 1A

FIG. 1B

FIG. 2

FIG. 3

FIG. 3A

FIG. 4

FIG. 5A

FIG. 5B

FIG. 6

FIG. 7

FIG. 8

FIG. 8A

FIG. 9

1010

Receiving a video stream

1020

Extracting images from the video stream to acquire images capturing at least a portion of a shape representative of a radiation pattern generated by a radiation delivery system that includes a radiation source configured to deliver the radiation beam

FIG. 10

FIG. 11

FIG. 12

FIG. 13

180

930

1400

930

940

FIG. 14

1510

Apply edge detection algorithm to emission pattern present in one or more images, the edge detection algorithm determining at least one edge of the emission pattern corresponding to a leaf of a multileaf collimator

1520

Determine a leaf position based at least on a location of the determined edge.

FIG. 15

FIG. 16

**EP 4 200 014 B1**

**REFERENCES CITED IN THE DESCRIPTION**

**Patent documents cited in the description**

- WO 2016200463 A1
- US 2008298553 A1
- US 2018140272 A1
- US 694865
- US 10617891 B
- US 395852